# EUROPÄISCHE PATENTSCHRIFT

(11) **EP 1 893 799 B1**
(45) Veröffentlichungstag und Bekanntmachung des Hinweises auf die Patenterteilung: **12.08.2015**
(21) Anmeldenummer: 06763719.9
(22) Anmeldetag: 14.06.2006
(51) Int. Cl.: B32B 5/02, D04H 1/4258, D04H 1/4391, D04H 1/492, D04H 1/498, A61F 13/15, B32B 5/26, D04H 1/435

(54) **IN WASSER AUFLÖSBARER BZW. ZERSETZBARER FASERVERBUND UND PRODUKTE DARAUS**
FIBRE COMPOSITE THAT CAN BE DISSOLVED OR DECOMPOSED IN WATER, AND PRODUCTS THEREOF
COMPOSITE FIBREUX POUVANT ETRE DISSOUS OU DECOMPOSE DANS L'EAU ET PRODUIT OBTENU A PARTIR DUDIT COMPOSITE

(30) Priorität: 15.06.2005 DE 102005027795; 23.06.2005 DE 102005029597
(43) Veröffentlichungstag der Anmeldung: 05.03.2008
(73) Patentinhaber: Kelheim Fibres GmbH, 93309 Kelheim (DE)
(72) Erfinder: BRUNNER, Konrad, 93309 Kelheim (DE); HERBIG, Friedrich, 93057 Regensburg (DE); ROGGENSTEIN, Walter, 93077 Bad Abbach (DE); SULZMAIER, Stefan, 93077 Bad Abbach (DE)
(74) Vertreter: Eisenführ Speiser
(86) Internationale Anmeldenummer: PCT/EP2006/063224
(87) Internationale Veröffentlichungsnummer: WO 2006/134132

(56) Entgegenhaltungen:
- JP-A- 8 003 848
- JP-A- 2004 293 027
- US-A- 3 131 693
- US-A- 3 171 773
- US-A- 3 370 590
- US-A- 5 068 141
- US-B1- 6 471 910
- DATABASE WPI Week 198735 Derwent Publications Ltd., London, GB; AN 1987-247412 XP002404856 & JP 62 170582 A (TEIJIN LTD) 27. Juli 1987 (1987-07-27) & JP 62 170582 A (TEIJIN LTD) 27. Juli 1987 (1987-07-27)

## Beschreibung

Die vorliegende Erfindung betrifft einen Faserverbund, umfassend oder bestehend aus natürlichen und/oder synthetischen Fasern, wobei zumindest ein Teil der Fasern des Verbundes einen flachen Querschnitt aufweist und der Faserverbund in Wasser und anderer Flüssigkeit leicht zersetzbar bzw. auflösbar ist. Die Erfindung betrifft auch aus diesem Faserverbund hergestellte bzw. diesen umfassende Produkte sowie ein Verfahren zur Herstellung eines solchen Faserverbundes. Die Erfindung betrifft insbesondere auch die Verwendung von Fasern mit flachem Querschnitt zur Verbesserung der Zersetzungs- bzw. Auflösungseigenschaften eines diese Fasern enthaltenden Faserverbundes in Wasser oder anderer Flüssigkeit.

### Stand der Technik

Wegwerf- bzw. Einwegartikel wie beispielsweise Feuchttücher zur Baby- bzw. Körperpflege, Babywindeln, Frauenhygieneprodukte etc. zeichnen sich im allgemeinen durch eine weiche und oftmals saugfähige Oberfläche sowie eine hohe Elastizität und Festigkeit sowie Strukturintegrität auch in Anwesenheit von Feuchtigkeit aus, so dass sie ihrem Zweck dienen können, ohne dabei kaputt zu gehen. Beispielsweise können Faserverbundstoffe (Vliesstoffe), welche die chemischen Fasern Polyester bzw. Polypropylen und Viskose enthalten, diese Materialeigenschaften aufweisen.

Bekannte derartige Polymermaterialien weisen jedoch den Nachteil auf, dass daraus hergestellte Verbundstrukturen sich nicht oder nicht ausreichend in Wasser zersetzen. Somit müssen sie über den normalen Hausmüll entsorgt werden und können nicht über gewöhnliche Hauskanalisationssysteme, beispielsweise durch Abspülen in der Toilette, entsorgt werden. Besonders in Ländern, in denen die Rohrleitungen eng verlegt sind, beispielsweise in Italien, bereiten Rohrverstopfungen durch nicht oder unzureichend lösbare Materialien, die in das System gelangen, große Probleme.

Eine vereinfachte Entsorgung über das Kanalisationssystem sowie eine insgesamt verbesserte biologische Abbaubarkeit von Polymermaterialien, die insbesondere für Körperpflegeprodukte wie Vliestücher und andere nichtgewobene Strukturen verwendet werden können, ist wünschenswert. Die Faserverbundstrukturen der Materialien sollen sich bereits im Abwasserkanal so weit zersetzen bzw. auflösen, dass sie in der Kläranlage nicht mehr erkennbar sind.

Es sind Veröffentlichungen bekannt, in denen temperatur- oder ionenempfindliche Zusammensetzungen für in Wasser dispergierbare oder abspülbare Materialien beschrieben sind. So beschreibt beispielsweise die DE 102 91 388 T1 in Wasser dispergierbare Polymere, Verfahren zu deren Herstellung und Gegenstände, in denen sie verwendet werden. Auch dort wird die Problematik der Entsorgbarkeit von Einwegartikeln wie Windeln, angefeuchteten Wischtüchern etc. beschrieben. Um die gewünschte Dispergierbarkeit der Artikel in kaltem Wasser zu erreichen, wird eine Zusammensetzung vorgeschlagen, die Natriumpolyacrylat und eine Styrolemulsion umfasst.

Die in EP 1 138 823 B1 beschriebene Methode, Vliesstrukturen ohne Probleme im Kanalisationssystem zu entsorgen, besteht darin, Fasermischungen mit definierter Faserlängenverteilung zu verwenden. Die Fasermischungen bestehen im wesentlichen aus Fasern mit einer maximalen Faserlänge von 10 mm sowie einem Anteil an Mikrofasern mit einer maximalen Faserlänge von 1 mm. Durch das Herauslösen der Mikrofasern in Anwesenheit von Wasser wird die Vliesstruktur zersetzt.

In EP 1 044 104 B1 erfolgt die Lösung der Aufgabenstellung durch die Verwendung eines in Wasser oder biologisch zersetzbarem Polymeres als Bestandteil der Vliesstruktur. Durch die Auflösung dieser spezifischen Polymerschicht im Entsorgungssystem zerfällt das Vlies in kleinere Bestandteile. Für manche Einsatzzwecke weisen diese Zusammensetzungen jedoch nicht die gewünschten Eigenschaften hinsichtlich Wasseraufnahmefähigkeit, Festigkeit, Zersetzbarkeit, biologische Abbaubarkeit o. dgl. auf.

JP 8-3848 beschreibt einen nichtgewebten Faserverbund, insbesondere einem durch Kardieren hergestellten Faserverbund, der bei niedrigen Temperaturen in Wasser auflösbar ist. Der Faserverbund besteht aus Fasern aus Polyvinylalkohol mit einer Schnittlänge von 51 mm und einem Titer von 2 Denier, die selbst wasserlöslich sind und ein bestimmter Anteil der PVA-Fasern (3-50 %, bezogen auf die Oberfläche des Faserverbunds) hat einen flachen Querschnitt mit einem Flachheitsverhältnis von 3-30.

US 3,313,693 beschreibt eine Windel, die in einem WC leicht zersetzbar und wegspülbar ist. Die Windel enthält einen papierartigen Faserverbund, der regenerierte Zellulosefasern mit bandförmiger, d.h. flacher, Struktur umfasst. Vorzugsweise haben diese Zellulosefasern eine Länge von 0,25 inch (6,3 cm), eine Breite von 0,12 mm und einen Titer von 1,5 den (1,7 dtex).

US 3,370,590 offenbart betrifft Faserverbunde aus Zellulosefasern, die sich auflösen, wenn sie in einem großen Überschuss an Wasser bewegt werden. Der Faserverbund enthält bandförmige flache Fasern aus regenerierter Zellulose.

JP 62-170582 offenbart mit Polyvinylalkohol (PVA) beschichtete Polyesterfasern für die Papierherstellung, die eine verbesserte Dispergierbarkeit in Wasser haben sowie eine gute Vereinbarkeit mit anderen Papierbestandteilen wie Zellstoffholz, Viskose und Bastfasern. Die mit PVA beschichteten Polyesterfasern haben einen Titer von 0,1 bis 10 denier, eine Länge von 3-30 mm und einen Querschnitt, der rund, sternförmig, dreieckig oder flach (insbesondere mit einem Flachheitsgrad von mindestens 2,5) sein kann.

### Aufgabenstellung

Eine Aufgabe der vorliegenden Erfindung besteht darin, einen Faserverbund und insbesondere ein Polymermaterial bzw. ein Polymergemisch in Faden- oder Vliesform zur Verfügung zu stellen, wobei der Faserverbund in Wasser, insbesondere in fließendem Wasser, ausreichend auflösbar und somit wegspülbar ist und gleichzeitig eine für seinen Verwendungszweck nötige Festigkeit und Elastizität, auch in Anwesenheit von Feuchtigkeit, aufweist.

Diese Aufgabe wird gelöst durch einen Faserverbund umfassend oder bestehend aus natürlichen und/oder synthetischen Fasern, wobei zumindest ein Teil der Fasern des Verbundes einen flachen Querschnitt aufweist, wobei die Fasern mit einem flachen Querschnitt gekennzeichnet sind durch eine Schnittlänge zwischen 4 und 75 mm, bevorzugt zwischen 20 und 50 mm und der Faserverbund in Wasser oder anderer Flüssigkeit leicht zersetzbar bzw. auflösbar ist, gekennzeichnet durch ein Breiten-Längenverhältnis der Seitenkanten eines Querschnittes der Fasern zwischen 1:4 und 1:6.Unter einem Faserverbund ist im Zusammenhang dieser Anmeldung ein Verbund mehrerer Einzelfasern zu verstehen. Dabei kann der Faserverbund aus Einzelfasern von mehr als einem Typus des gleichen Fasermaterials (z. B. Flach- und Rundfaser) und/oder aus Fasern aus unterschiedlichem Fasermaterial bestehen. Bevorzugt besitzt ein Faserverbund keine regelmäßige Struktur im Gegensatz z. B. von textilen Strukturen, die regelmäßige Fadenstrukturen aufweisen. Unter Flachfasern sind im Zusammenhang dieser Anmeldung Fasern zu verstehen, deren Querschnitt senkrecht zur Längsachse der jeweiligen Faser ein Seitenverhältnis von wenigstens 1:2 aufweist.

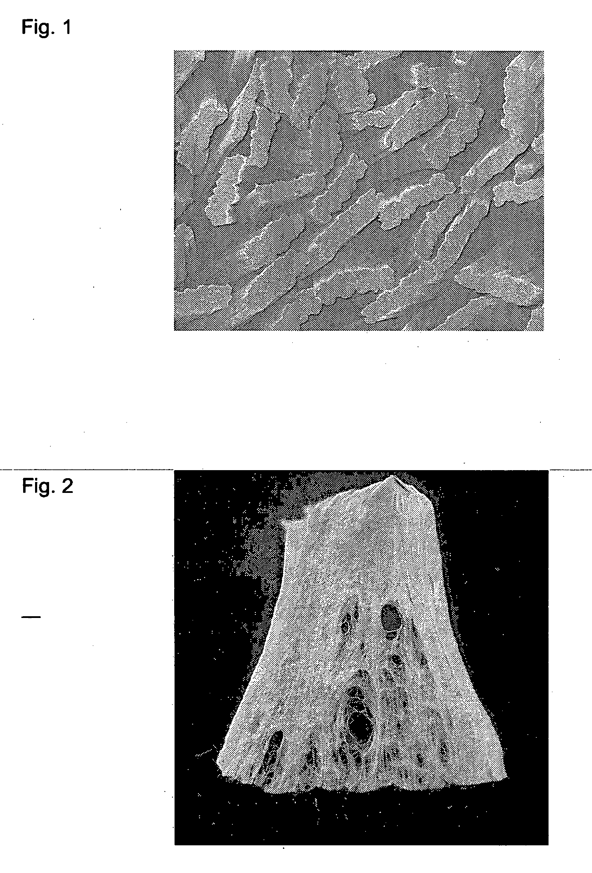

Unter "in Wasser oder anderer Flüssigkeit leicht zersetzbar bzw. auflösbar" ist im Zusammenhang dieser Anmeldung ein Faserverbund zu verstehen, der makroskopisch erkennbar deutliche Auflösungserscheinungen nach folgender Anzahl von Turns im nachfolgend beschriebenen "Flushability-Test" zeigt (vergleiche dazu auch Figur 2): 12 Turns bevorzugt 10 Turns, wiederum jeweils bevorzugt 9, 8, 7 und 6 Turns und ganz besonders bevorzugt 5 Turns. Der "Flushability-Test" zur Beurteilung der Zersetzbarkeit bzw. Auflösbarkeit des Faserverbundes simuliert die mechanische Belastung im Abwassersystem. Hierzu wird ein einseitig verschlossenes Kunststoffrohr mit einem Innendurchmesser von 100 mm und einer Länge von 1000 mm mit Wasser gefüllt, so dass sich eine Wassersäule von 330 mm ergibt. Eine Probe des zu untersuchenden Faserverbundes wird auf eine Dimension MD 220 mm und CD 110 mm (MD = Machine Direction, Längsrichtung und CD = Cross Direction, Querrichtung) vorbereitet und in das Rohr mit Wasser gegeben. Das mittig gelagerte und drehbare Kunststoffrohr wird mittels eines schraubbaren Deckels verschlossen. Durch Drehen des Rohres um jeweils 180 Grad wird eine Spülung simuliert. Die Drehungen werden solange vorgeführt, bis eine deutliche Öffnung des Fließverbandes mit teilweiser Riss- und Lochbildung zu erkennen ist, vergleiche Figur 2. Die Anzahl der 180-Grad-Drehungen (Turns) stellt das Messergebnis dar.

Dieser Test wird bevorzugt für die Zersetzbarkeit nicht-gewebter Strukturen verwendet.

Das Fasermaterial des erfindungsgemäßen Faserverbundes ist insbesondere ein Polymermaterial, das natürliche und/oder synthetische Fasern, bevorzugt Viskosefasern enthält. In einer besonders bevorzugten Variante ist ein Teil des Fasermaterials selbst in Wasser löslich.

Üblicherweise verwendete Viskose- oder Polymerfasern weisen einen annähernd kreisförmigen bzw. runden Querschnitt Im Gegensatz dazu weisen die im Zusammenhang mit der vorliegenden Erfindung verwendeten Fasern einen veränderten, insbesondere einen flachen, bevorzugt einen weitgehend rechteckförmigen und/oder trapezförmigen Querschnitt senkrecht zur Längsachse der Faser auf. Die einzelnen Fasern weisen somit eine bandartige Form auf.

Bevorzugt können die erfindungsgemäßen verwendeten Flachfasern zusätzlich eine krenellierte (zinnenartige) Oberfläche aufweisen. Eine derartige Oberflächenstruktur vergrößert die Oberfläche der Fasern und steigert deren Fähigkeit, Feuchtigkeit aufzunehmen. Die Saugfähigkeit der Endprodukte ist dadurch im Vergleich zu Fasern mit glatter Oberfläche erhöht. Die Flachfasern sind bevorzugt cellulosische Fasern, besonders bevorzugt Viskosefasern.

Der vorteilhafte Effekt, der bei der Verwendung der beschriebenen Flachfasern im erfindungsgemäßen Faserverbund festgestellt wurde, ist die stark verbesserte Auflösbarkeit bzw. Zersetzbarkeit des Faserverbundes in Wasser oder anderen Flüssigkeiten, was in der Praxis dazu führt, das derartige Faserverbunde bzw. daraus hergestellte Artikel als universelle Reinigungs- und Pflegeartikel verwendet werden können, die ohne nennenswerte Einschränkungen über herkömmliche Hauskanalisationssysteme entsorgt werden können. Dies wird als sog. "Flushability" (vergleiche oben) bezeichnet, da die Vliesstoffe über die Toilette weggespült werden können und in den Kanalisationssystemen und Kläranlagen weder Verstopfungen noch anderweitige Probleme verursachen. Aufgrund der verringerten Haftung der Flachfasem aneinander und an den im Vliesmaterial zusätzlich enthaltenen Rundfasern und/oder aufgrund des loseren Faserverbunds löst sich der Faserverbund in der Flüssigkeit deutlich schneller auf und wird dort in ausreichend kurzer Zeit in ausreichend kleine Verbundteile zersetzt.

Bevorzugt stellt der erfindungsgemäße Faserverbund ein nichtgewebtes Vlies oder einen Faden dar. Besonders bevorzugt ist dabei ein erfindungsmäßer Faserverbund, der durch ein Gemisch aus Fasermaterialien gekennzeichnet wird.

Die Fasern des erfindungsgemäßen Faserverbundes, der insbesondere Polymer- , Polyester-, Polypropylen-, Viskose- und/oder Cellulosefasern enthalten kann, können mit anderen Fasern, beispielsweise mit Polyesterfasern und/oder Polyethylenfasern in unterschiedlichen Mischverhältnissen vermengt zu bekannten Strukturen verarbeitet werden. Insbesondere können derartige Materialmischungen zu Faserverbundstoffen verarbeitet werden. Als solche kommen sie trocken oder befeuchtet zur Baby- bzw. Körperpflege, im Haushaltsbereich oder für andere Zwecke zur Anwendung. Eine Anwendbarkeit derartiger erfindungsgemäßer Materialien im hygienischen oder medizinischen Bereich (beispielsweise Frauenmonatshygiene, Wundverbände, etc.) ist ebenfalls gegeben. Auch die industrielle Verwendbarkeit von solchen Faserverbundstoffen ist vorteilhaft.

Der Anteil der Flachfasern am gesamten Fasermaterial kann in der Praxis grundsätzlich zwischen 1 Gew.-% und annähernd 100 Gew.-% liegen, wobei der Anteil der Flachfasern typischerweise zwischen 10 Gew.-% und 90 Gew.-% beträgt. Gute Eigenschaften können insbesondere mit einem Anteil der Flachfasern zwischen 20 Gew.-% und 80 Gew.-%, vorzugsweise zwischen 30 Gew.-% und 70 Gew.-% erreicht werden.

Besonders vorteilhafte Eigenschaften weist ein erfindungsgemäßer Faserverbund auf, der ein Polymergemisch ist, das zu 40 Gew.-% aus Polyester-Flachfasern (insb. PET) und zu 60 Gew.-% aus Viskoseflachfasern besteht. Die Viskosefasern verleihen der Vliesstruktur Weichheit und Saugfähigkeit, während die Polyester-Flachfasern insbesondere für die nötige Festigkeit sorgen.

Die Feinheit der im Faserverbund, insbesondere in Vliesen verwendeten Flachfasern kann typischerweise zwischen 0,9 und 9,0 dtex betragen, wobei die Feinheit bei einem erfindungsgemäßen Vlies vorzugsweise zwischen 1,5 und 3,0 dtex beträgt.

Die Schnittlänge der im erfindungsgemäßen Faserverbund verwendeten Flachfasern kann typischerweise zwischen 4 und 75 mm betragen, wobei die Schnittlänge bei einem erfindungsgemäßen Vliesstoff vorzugsweise zwischen 20 und 50 mm betragen kann.

Das spezifische Gewicht des erfindungsgemäßen Faserverbundes bzw. der in diesem verwendeten Flachfasern kann typischerweise zwischen 10 g/m² und 500 g/m² betragen, wobei das spezifische Gewicht bei einem erfindungsgemäßen Faserverbund besonders als Vlies vorzugsweise zwischen 40 und 150 g/m² beträgt. Besonders bevorzugt ist dabei ein spezifisches Gewicht zwischen 50 und 150 g/m².

In Laborversuchen zeigte eine nicht-gewebte (non-woven) bzw. im sog. Papierverfahren hergestellte Struktur, die einen erfindungsgemäßen Faserverbund enthielt oder darstellte, eine deutlich höhere Auflösungsrate in Wasser als Vergleichsstrukturen mit üblichen Viskosefasern mit kreisförmigem Querschnitt. Gleichzeitig konnte weder im trockenen noch im feuchten Zustand eine bedeutende Abnahme der Strukturfestigkeit gegenüber üblichen Vergleichsstrukturen festgestellt werden. Im Vergleich zu runden Viskosefasern bzw. runde Viskosefasern aufweisenden Faserverbundstoffen weisen die erfindungsgemäßen Viskose- bzw. Flachfasern bzw. erfindungsgemäße Viskose- oder Flachfasern aufweisende Faserverbundstoffe eine höhere Flexibilität und Weichheit auf.

Die in der Anwendung ebenfalls wichtigen Faktoren Feuchtigkeitsverhalten und Saugfähigkeit, insbesondere in der Mischung mit anderen Fasern, sind für die erfindungsgemäß eingesetzen Flachfasern ebenso hervorragend wie für bekannte gattungsgemäße Fasern.

Je nach Pigmentart und Pigmentanteil können die Fasern glänzend, mattiert oder farbig erscheinen.

Das Herstellungsverfahren erfindungsgemäßer Faserverbunde mit Viskose- oder Flachfasern entspricht dem bekannten Herstellungsverfahren für Faserverbunde und Faserverbundstoffe mit üblichen, runden Viskosefasern. Übliche Technologien sind beispielsweise, aber nicht ausschließlich die Wasserstrahlverfestigung, Airlaid-Verfahren oder die Vliesvernadelung. Die Technologie ist bekannt und bewährt, so dass die Herstellung der erfindungsgemäßen neuartigen Faserverbunde und der Artikel daraus sehr vereinfacht ist.

Das erfindungsgemäße Faserverbundmaterial ist vollständig biologisch abbaubar und nach Ökotex Standard Stufe 1 zertifizierbar sofern ausschließlich cellulosische Fasern, insbesondere Viskosefasern verwendet werden.

Teil der Erfindung ist es auch aus den erfindungsgemäßen Faserverbunden oder unter ihrer Verwendung Artikel (Zwischen- oder Endprodukte) für bestimmte Verwendungen herzustellen. Dazu gehören Vliesstoffe und Fasersubstrate, im Haushalt und/oder zur Reinigung verwendbare Wischtücher, saugfähige Vliestücher, Hygienetücher oder Baby- und/oder Körperpflegetücher. Auch Artikel zur technischen Anwendung können aus dem erfindungsgemäßen Faserverbund oder unter seiner Verwendung hergestellt werden. Zu solchen Artikeln gehören zum Beispiel ein technischer Vliesstoff bzw. technisches Vliesmaterial und ein Filterstoff bzw. Filtermaterial.

Ein besonders bevorzugter erfindungsgemäßer Artikel ist ein Tuch für verschiedene Anwendungen, das mindestens zwei Schichten umfasst, wobei eine der Schichten im Wesentlichen aus einem erfindungsgemäßen Faserverbund besteht. So hat man für bestimmte Anwendungen beispielsweise eine besonders leicht auflösbare Schicht, während die andere Schicht - falls dies gewünscht ist - weniger leicht auflösbar bzw. zersetzbar ist als der erfindungsgemäße Faserverbund. Bevorzugt ist jedoch, dass auch die weiteren Schichten zumindest hinsichtlich ihrer Struktur ebenfalls leicht zersetzbar bzw. auflösbar sind. Ganz besonders bevorzugt ist in diesem Zusammenhang, dass wenigstens eine weitere Schicht aus Cellulosefaserpulp besteht.

Die Kombination einer Schicht aus erfindungsgemäßem Faserverbund und einer weiteren Schicht aus Cellulosepulp zu Tüchern führt dazu, dass Tücher hergestellt werden, die über eine gute Flushability verfügen (d. h. deren Faserverbundstrukturen sich ausreichend und gut in Kanalisationssystemen auflösen). Gleichzeitig besitzen solche Tücher eine rauhe Seite (Cellulosepulp) mit entsprechenden Wisch- und Saugeigenschaften und eine weiche Seite mit von der rauhen Seite abweichenden Eigenschaften. So kann die rauhe Seite beispielsweise beim Einsatz als Pflegetuch in der Babypflege zur Aufnahme grober Verunreinigungen verwendet werden, während die weiche Seite, die den erfindungsgemäßen Faserverbund umfasst, zur Feinreinigung mit viel Hautkontakt verwendet werden kann.

Bestandteil der Erfindung ist auch die Verwendung von Fasern mit einem flachen Querschnitt zur Verbesserung der Zersetzungs- bzw. Auflösungseigenschaften eines diese Fasern enthaltenden Faserverbundes in Wasser oder anderer Flüssigkeit.

Eine erfindungsgemäße Verwendung von Flachfasern insbesondere Viskoseflachfasern in der Textilindustrie, auch in Kombination mit anderen als den genannten Materialien und in anderen Strukturen (beispielsweise Garne, Gewebe, Gewirke etc.), ist denkbar.

Bestandteil der Erfindung ist auch die Herstellung eines erfindungsgemäßen Faserverbundes umfassend die Schritte
a) Bereitstellen von Flachfasern,
b) gegebenenfalls Bereitstellen von weiteren Fasern und
c) Verbinden der Fasern zu einem Faserverbund, so dass der Verbund in Wasser oder anderer Flüssigkeit leicht zersetzbar bzw. auflösbar ist.

### Ausführungsbeispiele

Die Erfindung wird nachfolgend anhand bevorzugter Ausführungsbeispiele unter Bezugnahme auf die beiliegenden Figuren näher erläutert.

Figur 1 zeigt einen vergrößerten Querschnitt der Faserstruktur eines erfindungsgemäßen Faserverbundes. Die einzelnen Viskose- bzw. Flachfasern sind hier sichtbar und es wird deutlich, dass diese bandartig verlaufen. Ihr Querschnitt senkrecht zur Längsachse der Faser ist flach. Die Oberflächen der einzelnen Viskosefasern weisen jeweils in Längsrichtung langgestreckte Furchen auf, so dass der Querschnitt der einzelnen Faser keinen glatten, sondern einen krenellierten (zinnenartigen) Randverlauf aufweist.

Figur 2 verdeutlicht den Beginn einer sich zersetzenden Faserverbundstruktur hier einer Vliesstruktur.

### Beispiel 1

Aus einer Viskoseflachfaser mit einer Faserfeinheit von 2,4 dtex und einer Faserlänge von 38 mm wurde mittels einer dem Stand der Technik entsprechenden Wasservernadelungsanlage bei einem Wasserdruck von 50 bar und einer Transportgeschwindigkeit von 70 m/min eine Vliesstruktur mit einem Flächengewicht von 50 g/m² hergestellt. Bei einem nach Beschreibung durchgeführten Flushability-Test wurde nach nur 7 Umdrehungen eine Vlieszersetzung beobachtet. Es wurden Zugfestigkeiten von MD Trocken 41,8 N/5cm und CD Trocken von 12,9 N/5cm gemessen. Eine vergleichbare Vliesstruktur aus einer Viskosefaser mit annähernd kreisförmigem Querschnitt, einer Faserfeinheit von 1,7 dtex und einer Schnittlänge von 38 mm, hergestellt mit einer dem Stand der Technik entsprechenden Wasservernadelungsanlage bei einem Wasserdruck von 50 bar und einer Transportgeschwindigkeit von 50 m/min zeigte bei einem Flächengewicht von 50 g/m² eine erste Vlieszersetzung nach erst 12 Drehungen.

### Beispiel 2

Eine Viskoseflachfaser mit einer Faserfeinheit von 2,4 dtex und einer Schnittlänge von 38 mm wurde wie im Beispiel 1 beschrieben mit einem Wasserdruck von 50 bar und einer Transportgeschwindigkeit von 100 m/min zu einer Vliesstruktur mit einem Flächengewicht von 50 g/m² verarbeitet. Die erste Vlieszersetzung wurde entsprechend der beschriebenen Flushability Testmethode nach bereits 5 Drehungen beobachtet.

### Beispiel 3

Bei einer Vliesstruktur, hergestellt in einem den Beispielen 1 und 2 vergleichbaren Verfahren unter Verwendung einer Viskoseflachfaser mit einer Faserfeinheit von 2,4 dtex und einer Schnittlänge von 38 mm in einer 50% / 50% Mischung mit einer Viskosefaser, mit annähernd kreisförmigem Querschnitt, einer Faserfeinheit von 1,7 dtex und einer Schnittlänge von 38 mm wurde die nach dem beschriebenen Flushability Test erste Vlieszersetzung nach 7 Drehungen festgestellt.

### Beispiel 4

Eine Vliestruktur, bestehend aus der in Beispiel 1 beschriebenen Viskoseflachfaser mit einem Gewichtsanteil am Gesamtvlies von 65 % als einer ersten Schicht und cellulosischem Pulpmaterial mit einem Gewichtsanteil am Gesamtvlies von 35 % als einer zweiten Schicht zeigte in dem beschriebenen Testverfahren eine erste Vliesauflösung nach 10 Drehungen. Die Schichten waren durch Wasserstrahlverfestigung verbunden worden.

## Patentansprüche

1. Faserverbund, umfassend oder bestehend aus natürlichen und/oder synthetischen Fasern, wobei zumindest ein Teil der Fasern des Verbundes einen flachen Querschnitt aufweist, wobei die Fasern mit einem flachen Querschnitt **gekennzeichnet sind durch** eine Schnittlänge zwischen 4 und 75 mm, bevorzugt zwischen 20 und 50 mm und der Faserverbund in Wasser oder anderer Flüssigkeit leicht zersetzbar bzw. auflösbar ist, **gekennzeichnet durch** ein Breiten-Längenverhältnis der Seitenkanten eines Querschnittes der Fasern zwischen 1:4 und 1:8.

2. Faserverbund nach Anspruch 1, **gekennzeichnet durch** einen rechteckförmigen und/oder trapezförmigen Querschnitt zumindest eines Teils der Fasern.

3. Faserverbund nach Anspruch 1 oder 2, umfassend oder bestehend aus Viskose- und/oder Cellulosefasern.

4. Faserverbund nach einem der einem der vorangehenden Ansprüche, umfassend oder bestehend aus Polymerfasern, insbesondere Polyester- und/oder Polypropylenfasern.

5. Faserverbund nach einem der einem der vorangehenden Ansprüche, **dadurch gekennzeichnet, dass** er ein nicht gewebtes Vlies oder ein Faden ist.

6. Faserverbund nach einem der vorangehenden Ansprüche, **gekennzeichnet durch** ein Gemisch aus verschiedenen Fasermaterialien.

7. Faserverbund nach einem der vorangehenden Ansprüche, **gekennzeichnet durch** eine Feinheit der Flachfasern zwischen 0,9 und 9,0 dtex. bevorzugt zwischen 1,5 und 3,0 dtex.

8. Faserverbund nach einem der vorangehenden Ansprüche, **gekennzeichnet durch** ein spezifisches Gewicht.zwischen 10 g/m² und 500 g/m², bevorzugt zwischen 40 g/m² und 150 g/m².

9. Faserverbund nach einem der vorangehenden Ansprüche, **gekennzeichnet durch** einen Anteil der Flachfasern zwischen 1 und 100 Gew-%, bevorzugt zwischen 10 und Gew.-90%.

10. Vliesstoff bzw. Fasersubstrat, bestehend aus oder umfassend einen Faserverbund gemäß einem der vorangehenden Ansprüche.

11. Im Haushalt und/oder zur Reinigung verwendbares Wischtuch, saugfähiges Vliestuch, Hygienetuch oder Baby- und/oder Körperpflegetuch, bestehend aus oder umfassend einen Faserverbund gemäß einem Ansprüche 1 bis 9.

12. Technischer Vliesstoff bzw. technisches Vliesmaterial oder Filterstoff bzw. Filtermaterial, bestehend aus oder umfassend einen Faserverbund gemäß einem Ansprüche 1 bis 9.

13. Tuch nach Anspruch 11, umfassend mindestens zwei Schichten, wobei eine der Schichten im Wesentlichen aus einem Faserverbund gemäß einem Ansprüche 1 bis 9 besteht.

14. Tuch nach Anspruch 13, wobei die weitere Schichte oder die weiteren Schichten ebenfalls leicht zersetzbar bzw. auflösbar sind.

15. Tuch nach Anspruch 14, wobei wenigstens eine weitere Schicht aus Pulp aus Cellulosefasern besteht.

16. Verwendung von Fasern mit einem flachen Querschnitt und einer Schnittlänge zwischen 4 und 75 mm, bevorzugt zwischen 20 und 50 mm **gekennzeichnet durch** ein Breiten-Längenverhältnis der Seitenkanten eines Querschnittes der Fasern zwischen 1:4 und 1:8, zur Verbesserung der Zer-setzungs- bzw. Auflösungseigenschaften eines diese Fasern enthaltenden Faserverbundes in Wasser oder anderer Flüssigkeit.

17. Verfahren zur Herstellung eines Faserverbundes nach einem der Ansprüche 1 bis 9 umfassend die Schritte:
a) Bereitstellen von Flachfasern mit einer Schnittlänge zwischen 4 und 75 mm, bevorzugt zwischen 20 und 50 mm,
b) gegebenenfalls Bereitstellen von weiteren Fasern und
c) Verbinden der Fasern zu einem Faserverbund, so dass der Verbund in Wasser oder anderer Flüssigkeit leicht zersetzbar bzw. auflösbar ist.

## Claims

1. Fibrous composite comprising or consisting of natural and/or synthetic fibres, **characterised in that** at least some of the fibres of the composite exhibit a flat cross-section, wherein the fibres with a flat cross section are **characterised by** a cut length of the flat fibres between 4 and 75 mm, preferably between 20 and 50 mm and the fibrous composite is readily decomposable or dissoluble in water or other liquid, **characterised by** a width/length ratio of the lateral edges of a cross-section of the fibres between 1:4 and 1:8.

2. Fibrous composite according to Claim 1, **characterised by** a rectangular and/or trapezoidal cross-section of at least some of the fibres.

3. Fibrous composite according to Claim 1 or 2, comprising or consisting of viscose fibres and/or cellulose fibres.

4. Fibrous composite according to one of the preceding claims, comprising or consisting of polymer fibres, in particular polyester fibres and/or polypropylene fibres.

5. Fibrous composite according to one of the preceding claims, **characterised in that** it is a non-woven fleece or a thread.

6. Fibrous composite according to one of the preceding claims, **characterised by** a mixture of various fibrous materials.

7. Fibrous composite according to one of the preceding claims, **characterised by** a fineness of the flat fibres between 0.9 and 9.0 dtex, preferably between 1.5 and 3.0 dtex.

8. Fibrous composite according to one of the preceding claims, **characterised by** a specific weight between 10 g/m² and 500 g/m², preferably between 40 g/m² and 150 g/m².

9. Fibrous composite according to one of the preceding claims, **characterised by** a proportion of the flat fibres between 1 wt.% and 100 wt.%, preferably between 10 wt.% and 90 wt.%.

10. Fleece material or fibre substrate consisting of or comprising a fibrous composite according to one of the preceding claims.

11. Wiping cloth that is capable of being used in the home and/or for cleansing, absorbent fleece cloth, hygienic cloth or baby cloth and/or personal-hygiene cloth, consisting of or comprising a fibrous composite according to one of Claims 1 to 9.

12. Technical fleece fabric or technical fleece material or filter fabric or filter material, consisting of or comprising a fibrous composite according to one of Claims 1 to 9.

13. Cloth according to Claim 11, comprising at least two layers, one of the layers consisting substantially of a fibrous composite according to one of Claims 1 to 9.

14. Cloth according to Claim 13, wherein the further layer or layers are likewise readily decomposable or dissoluble.

15. Cloth according to Claim 14, wherein at least one further layer of pulp consists of cellulose fibres.

16. Use of fibres with a flat cross-section and a cut length of between 4 and 75 mm, preferably between 20 and 50 mm, **characterised by** a width/length ratio of the lateral edges of a cross-section of the fibres between 1:4 and 1:8 for the purpose of improving the decomposition properties or dissolution properties in water or other liquid of a fibrous composite containing these fibres.

17. Process for producing a fibrous composite according to one of Claims 1 to 9, comprising the following steps:
a) providing flat fibres with a cut length of between 4 and 75 mm, preferably between 20 and 50 mm,
b) optionally, providing further fibres and
c) connecting the fibres to form a fibrous composite, so that the composite is readily decomposable or dissoluble in water or other liquid.

## Revendications

1. Assemblage de fibres comprenant des fibres naturelles et/ou synthétiques ou constitué de fibres naturelles et/ou synthétiques, dans lequel au moins une partie des fibres de l'assemblage présente une section transversale plate, dans lequel les fibres présentant une section transversale plate sont **caractérisées par** une longueur de coupe de 4 à 75 mm, de préférence de 20 à 50 mm, et l'assemblage de fibres étant facilement soluble ou décomposable dans l'eau ou autre liquide, **caractérisé en ce que** le rapport largeur-longueur des bords latéraux d'une section transversale des fibres est compris entre 1 : 4 et 1 : 8.

2. Assemblage de fibres selon la revendication 1, **caractérisé par** une section transversale rectangulaire et/ou trapézoïdale d'au moins une partie des fibres.

3. Assemblage de fibres selon la revendication 1 ou 2, comprenant ou consistant en fibres de viscose et/ou fibres de cellulose.

4. Assemblage de fibres selon l'une quelconque des revendications précédentes, comprenant des fibres polymères ou constitué de fibres polymères, en particulier de fibres de polyester et/ou de fibres polypropylènique.

5. Assemblage de fibres selon l'une quelconque des revendications précédentes, **caractérisé en ce qu'**il consiste en un non-tissé ou un fil.

6. Assemblage de fibres selon l'une quelconque des revendications précédentes, **caractérisé en ce qu'**il consiste en un mélange de différents matériaux fibreux.

7. Assemblage de fibres selon l'une quelconque des revendications précédentes, **caractérisée par** une finesse de fibres de 0,9 à 9,0 dtex, de préférence de 1,5 à 3,0.

8. Assemblage de fibres selon l'une quelconque des revendications précédentes, **caractérisé par** un poids spécifique compris entre 10 g/m² et 500 g/m², de préférence entre 40 g/m² et 150 g/m².

9. Assemblage de fibres selon l'une quelconque des revendications précédentes, **caractérisé par** une proportion de fibres plates comprise entre 1 et 100% en poids, de préférence entre 10 et 90% en poids.

10. Non tissé ou substrat de fibres, comprenant ou constitué d'un assemblage de fibres selon l'une quelconque des revendications précédentes.

11. Chiffon utilisable pour le ménage ou le nettoyage, non-tissés absorbants ou lingettes hygiéniques et/ou lingettes pour les bébés et/ou pour soins corporels, comprenant un assemblage de fibres ou constitué d'un assemblage de fibres selon l'une quelconque des revendications 1 à 9.

12. Non-tissé technique, matériau non-tissé technique, matière filtrante ou matériau filtrant comprenant un assemblage de fibres ou constitué d'un assemblage de fibres selon l'une quelconque des revendications 1 à 9.

13. Lingette selon la revendication 11, comprenant au moins deux couches, dans laquelle l'une des couches consiste essentiellement en un assemblage de fibres selon l'une des revendications 1 à 9.

14. Lingette selon la revendication 13, dans laquelle l'autre couche ou les autres couches sont également facilement décomposables ou solubles.

15. Lingette selon la revendication 14, dans laquelle au moins une autre couche est constituée de pulpe de fibres de cellulose.

16. L'utilisation de fibres ayant une section transversale plate et une longueur de coupe de 4 à 75 mm, de préférence de 20 à 50 mm, **caractérisées en ce que** le rapport largeur-longueur des bords latéraux d'une section transversale des fibres est compris entre 1 : 4 et 1 : 8.et l'assemblage de fibres pour améliorer les propriétés de décomposition ou les propriétés de solubilisation d'un des assemblages de fibres précités dans l'eau ou autre liquide

17. Procédé de production d'un assemblage de fibres selon l'une des revendications 1 à 9, comprenant les étapes consistant à:
a) mettre à disposition un assemblage de fibres plates dont la longueur de coupe est de 4 à 75 mm, de préférence de 20 à 50 mm,
b) le cas échéant mettre à disposition d'autres fibres et
c) lier les fibres pour former un assemblage de fibres tel que la liaison soit facilement décomposable ou dissoluble dans l'eau ou autre liquide.
